# EUROPÄISCHE PATENTSCHRIFT

(11) **EP 4 331 723 B1**
(45) Veröffentlichungstag und Bekanntmachung des Hinweises auf die Patenterteilung: **18.06.2025**
(21) Anmeldenummer: 22193653.7
(22) Anmeldetag: 02.09.2022
(51) Int. Cl.: B01L 3/00, C12M 1/32, B29C 65/58, C12M 1/00

(54) **SET UMFASSEND EINE MIKROPLATTE, EINEN DECKEL UND EINE VORRICHTUNG ZUM LÖSEN EINER SCHNAPPVERBINDUNG ZWISCHEN MIKROPLATTE UND DECKEL**
SET COMPRISING A MICROPLATE, A LID AND A DEVICE FOR RELEASING A SNAP CONNECTION BETWEEN MICROPLATE AND LID
ENSEMBLE COMPRENANT UNE MICROPLAQUE, UN COUVERCLE ET UN DISPOSITIF DE LIBÉRATION D'UNE LIAISON ENCLIQUETABLE ENTRE LA MICROPLAQUE ET LE COUVERCLE

(43) Veröffentlichungstag der Anmeldung: 06.03.2024
(73) Patentinhaber: Eppendorf SE, 22339 Hamburg (DE)
(72) Erfinder: Schwarzwald, Detlef, 22417 Hamburg (DE)
(74) Vertreter: Hauck Patent- und Rechtsanwälte PartmbB

(56) Entgegenhaltungen:
- DE-B4- 10 066 431
- US-A1- 2003 180 191
- US-A1- 2021 299 655

## Beschreibung

Die Erfindung betrifft ein Set umfassend eine Mikroplatte, auch Mikrotiterplatte, Mikrotestplatte oder Multiwell-Platte genannt, einen Deckel zum Abdecken der Mikroplatte und eine Vorrichtung zum Lösen einer Schnappverbindung zwischen Mikroplatte und Deckel.

Mikroplatten werden insbesondere in wissenschaftlichen und industriellen Laboren mit medizinischen, molekularbiologischen oder pharmazeutischen Anwendungsgebieten für die unterschiedlichsten mikrobiologischen, zellbiologischen und immunologischen Arbeitsgänge benutzt. Beispielsweise finden Mikroplatten Anwendung in der PCR und der Züchtung von Mikroorganismen oder Zellen.

Mikroplatten haben einen Rahmen mit einer Vielzahl Gefäßen (Wells) zur Aufnahme von Probenflüssigkeit. Bekannt sind Ausführungsarten, bei denen die Gefäße als Vertiefungen in einem insgesamt plattenförmigen Rahmen ausgebildet sind. Bei anderen Ausführungsarten sind die Gefäße oben mit einer Platte des Rahmens verbunden, stehen von der Unterseite der Platte nach unten vor und weisen an der Oberseite der Platte eine Öffnung auf. Der äußere Rand der Platte kann mit einer nach unten vorstehenden, umlaufenden Seitenwand des Rahmens verbunden sein. Die Seitenwand kann weiter nach unten von der Platte vorstehen, als die Gefäße (*skirted*). Ferner sind Ausführungen bekannt, bei denen die Seitenwand weniger weit als die Gefäße nach unten von der Platte vorsteht (*semi-skirted*). Es gibt aber Ausführungen, bei denen die Platte nicht mit einer Seitenwand verbunden ist (*unskirted*). Bekannte Mikroplatten mit Seitenwand weisen am unteren Rand der Seitenwand eine Erweiterung auf, mit der sie auf die Oberseite einer weiteren Mikroplatte aufsetzbar sind. Mikroplatten mit großen Gefäßen werden auch als "Deepwell-Platten" bezeichnet.

Mikroplatten sind durch die Standards der ANSI/SLAS für Mikroplatten insbesondere hinsichtlich der Grundfläche (*footprint*) und der Anordnung der Gefäße bei Mikroplatten mit 96, 384 und 1.536 Gefäßen standardisiert. Besonders verbreitet sind Mikroplatten mit 96 Gefäßen, bei denen die Gefäße in acht Reihen und zwölf Spalten jeweils einen Abstand von 9 mm voneinander aufweisen.

Bekannt sind auch Mikroplatten und Deepwell-Platten mit 48 Gefäßen in sechs Reihen und acht Spalten, die beispielsweise für Zellkulturen verwendet werden. Diese Mikroplatten werden mit derselben Grundfläche bzw. Länge und Breite wie die Mikroplatten mit 96 Gefäßen gemäß den Standards der ANSI/SLAS für Mikroplatten angeboten.

Die DE 100 66 431 B4 beschreibt eine Mikroplatte aus Kunststoff mit einem auf der Oberseite der Platte lösbar anbringbaren starren Deckel und mindestens einer Dichtung zwischen Deckel und Platte aus einem elastischen Material. Bei einem Ausführungsbeispiel stehen propfenförmige Dichtungen von der Unterseite der Platte vor, die in Öffnungen der Gefäße eingreifen, so dass sie mit Dichtwulsten an ihrem Außenumfang abdichtend an der Innenwand der Gefäße anliegen, wenn der Deckel auf die Mikroplatte aufgesetzt ist. Der Deckel hat vom Rand der Platte nach unten vorstehende Einfassungen, von denen nach innen Rastvorsprünge vorstehen, welche in Ausnehmungen der Seitenwand unterhalb der Platte der Mikroplatte einrastbar sind. Die Einfassungen des Deckels haben nach oben vorstehende Handgriffe, die das Ergreifen des Deckels erleichtern. Durch Schwenken der Handgriffe kann die Verrastung zwischen den Rastvorsprüngen und den Ausnehmungen aufgelöst werden, weil die Einfassungen mitgeschwenkt werden. Für ein automatisiertes Plattenhandling sind die Mikroplatte und der Deckel insbesondere wegen der Auflösung der Schnappverbindung mittels der Handgriffe nicht geeignet.

Davon ausgehend liegt der Erfindung die Aufgabe zugrunde, eine Technik zur Verfügung zu stellen, die ein einfaches Auflösen einer Schnappverbindung zwischen Mikroplatte und Deckel ermöglicht, die auch in einem automatisierten Prozess durchgeführt werden kann.

Die Aufgabe wird durch ein Set gemäß Anspruch 1 gelöst. Vorteilhafte Ausführungsarten des Sets sind in Unteransprüchen und in der nachfolgenden Beschreibung angegeben.

Das erfindungsgemäße Set umfasst:
- eine Mikroplatte,
- mit einer rechteckigen ersten Platte,
- einer Vielzahl Gefäße, die in Reihen und Spalten angeordnet sind, oben mit der ersten Platte verbunden sind, von der Unterseite der ersten Platte nach unten vorstehen und an der Oberseite der ersten Platte eine Öffnung aufweisen, und
- Schnappöffnungen an mindestens zwei einander gegenüberliegenden seitlichen Rändern der ersten Platte,
- einen Deckel,
- mit einer zweiten Platte zum Aufsetzen auf die Oberseite der ersten Platte und Abdecken der Öffnungen der Gefäße und
- von der Unterseite der zweiten Platte vorstehenden Schnapphaken an mindestens zwei einander gegenüberliegenden seitlichen Rändern der zweiten Platte, wobei der Deckel in einer Abdeckposition mit der zweiten Platte auf die Oberseite der ersten Platte aufsitzt, die Öffnungen der Gefäße abdeckt und mit den Schnapphaken unter Ausbildung von Schnappverbindungen in die Schnappöffnungen eingeschnappt ist, und
- eine Entriegelungsvorrichtung zum Lösen der Schnappverbindungen zwischen Mikroplatte und Deckel,
- mit mindestens zwei hochstehenden Stützvorsprüngen und mindestens einer Aufnahme dazwischen zum Aufsetzen einer Mikroplatte mit mindestens zwei einander gegenüberliegenden Randbereichen der ersten Platte auf die Stützvorsprünge unter Eingriff der Gefäße in die mindestens eine Aufnahme und
- erste Gleitflächen an einander zugewandten Innenseiten der Schnapphaken an einander gegenüberliegenden Rändern der ersten Platte und zweite Gleitflächen an voneinander abgewandten Außenseiten der Stützvorsprünge auf einander gegenüberliegenden Seiten der Aufnahme, wobei die ersten Gleitflächen an einander zugewandten Innenseiten der Schnapphaken nach oben aufeinander zugeneigt sind und/oder die zweiten Gleitflächen an voneinander abgewandten Außenseiten der Stützvorsprünge nach oben aufeinander zugeneigt sind und beim Aufsetzen der Mikroplatte mit dem aufgeschnappten Deckel auf die Entriegelungsvorrichtung durch Abgleiten der zweiten Gleitflächen auf den ersten Gleitflächen die Schnapphaken nach außen ausgelenkt und die Schnappverbindungen aufgehoben werden.

Bei dem erfindungsgemäßen Set wird der Deckel von oben auf die Mikroplatte aufgesetzt, um die Öffnungen der Gefäße abzudecken und die in Gefäße eingefüllte Flüssigkeit vor Verlust bzw. Kontamination zu schützen. Hierbei tauchen die Schnapphaken in die Schnappöffnungen ein, bis sie an die Schnappöffnungen angrenzende Randbereiche der ersten Platte hintergreifen, wodurch Schnappverbindungen zwischen Mikroplatte und Deckel gebildet werden. Der Deckel kann wieder von der Mikroplatte gelöst werden, indem die Mikroplatte mit dem damit verschnappten Deckel auf die Entriegelungsvorrichtung zum Lösen der Schnappverbindungen ("Entriegelungsvorrichtung"), aufgesetzt wird. Beim Aufsetzen der Mikroplatte mit dem aufgeschnappten Deckel auf die Entriegelungsvorrichtung werden durch Abgleiten der zweiten Gleitflächen auf den ersten Gleitflächen die Schnapphaken nach außen ausgelenkt und die Schnappverbindungen aufgehoben. Allein durch Aufsetzen der durch den Deckel verschlossenen Mikroplatte auf die Entriegelungsvorrichtung werden die Schnappverbindungen wieder gelöst und der Deckel kann in einer weiteren Bewegung von der Mikroplatte abgehoben werden.

Bei dem erfindungsgemäßen Set können die Schnappverbindungen zwischen Mikroplatte und Deckel einfach hergestellt werden. Ferner können die Schnappverbindungen einfach aufgehoben werden, indem die Mikroplatte zusammen mit dem darin aufgeschnappten Deckel auf die Entriegelungsvorrichtung aufgesetzt wird. Ferner ist es möglich, den Deckel automatisiert zu handhaben. Das Verschließen und Verschnappen der Mikroplatte durch den Deckel kann durch einfaches Aufsetzen des Deckels auf die Mikroplatte mittels eines Automaten erfolgen. Das Auflösen der Verschnappung kann durch einfaches Aufsetzen der durch den Deckel abgedeckten Mikroplatte auf die Entriegelungsvorrichtung mittels eines Automaten erfolgen. Auch ist eine manuelle Handhabung möglich.

Ein weiterer Vorteil ist, dass vorhandene Mikroplatten ohne Veränderungen für das Set verwendet werden können. Das Set kann sowohl mit vorhandenen Mikroplatten als auch mit angepassten Mikroplatten und Deepwell-Platten verwendet werden. Das Herstellen der Schnappverbindungen und das Auflösen der Schnappverbindungen ist sowohl eine automatisierte Durchführung mittels eines Laborautomaten oder Dosierautomaten oder für eine manuelle Durchführung durch Laborpersonal geeignet.

Gemäß einer Ausführungsart der Erfindung ist jede Schnappverbindung zumindest teilweise in den beiden seitlichen Rändern der ersten Platte angeordnet. Hierdurch wird das Lösen der Schnappverbindung durch Auslenken der Schnapphaken nach außen erleichtert, weil die Schnapphaken durch Eintreten ihrer Enden in die Schnappöffnungen einer ersten Platte freikommen.

Die Schnappöffnungen können ausschließlich in den seitlichen Rändern der ersten Platte ausgebildet sein, insbesondere bei einer Mikroplatte, die keine Seitenwand aufweist. Auch kann eine Mikroplatte mit Seitenwand die Schnappöffnungen ausschließlich in der ersten Platte aufweisen, wobei die Schnappöffnungen groß genug ausgebildet sein müssen, die Schnapphaken ausgelenkt werden können, bis sie die erste Platte nicht mehr untergreifen und aus den Schnappöffnungen herausgezogen werden können.

Gemäß einer weiteren Ausführungsart umfasst die Mikroplatte eine vom äußeren Rand der ersten Platte nach unten vorstehende, umlaufende Seitenwand und ist jede Schnappöffnung zumindest teilweise im oberen Randbereich der Seitenwand ausgebildet. Durch die zumindest teilweise Ausbildung jeder Schnappöffnung im oberen Randbereich der Seitenwand wird das Auflösen der Schnappverbindungen durch seitliches Auslenken der Schnapphaken begünstigt.

Bei einer bevorzugten Ausführungsart ist jede Schnappöffnung zu einem Teil im seitlichen Randbereich der ersten Platte und zu einem weiteren Teil im oberen Randbereich der Seitenwand angeordnet. Dieser Ausführungsart kommen die obigen Vorteile in Kombination zu.

Die Schnappöffnungen können auch ausschließlich in der Seitenwand ausgebildet sein.

Ferner wird durch Ausbildung der Schnappöffnungen sowohl an den Rändern der ersten Platte als auch in der Seitenwand das Entformen der Mikroplatte aus dem Spritzgieß-Werkzeug bei der Herstellung durch Spritzgießen erleichtert.

Gemäß einer weiteren Ausführungsart weist der Teil der Schnappöffnungen, der im seitlichen Randbereich der ersten Platte ausgebildet ist, eine parallel zum äußeren Rand der ersten Platte verlaufende obere Begrenzungsfläche auf, die mit der Innenseite der Seitenwand fluchtet oder bezüglich dieser nach innen versetzt ist, die an eine untere Begrenzungsfläche des Teils der Schnappöffnung im oberen Randbereich der Seitenwand angrenzt. Hierdurch wird das Entformen der Mikroplatte aus dem Spritzgießwerkzeug bei der Herstellung durch Spritzgießen erleichtert.

Gemäß einer weiteren Ausführungsart weist die Mikroplatte an den beiden langen Rändern der ersten Platte jeweils zwei Schnappöffnungen auf und weist der Deckel an den beiden langen Rändern der zweiten Platte jeweils zwei Schnapphaken auf. Hierdurch wird eine gleichmäßige abdichtende Anlage des Deckels an der Mikroplatte begünstigt.

Gemäß einer weiteren Ausführungsart weist die Mikroplatte an den beiden kurzen Rändern jeweils zwei Schnappöffnungen auf und weist der Deckel an den beiden kurzen Rändern jeweils zwei Schnapphaken auf. Hierdurch wird die gleichmäßige Anlage des Deckels an der Mikroplatte weiter gefördert.

Gemäß einer weiteren Ausführungsart weisen die Schnapphaken einen vertikalen Steg und am unteren Ende des vertikalen Steges einen nach innen vorstehenden horizontalen Steg auf. Hierdurch werden flexible Schnapphaken erreicht, die ein einfaches Einschnappen in die Schnappöffnungen und ein einfaches gezieltes Lösen ermöglichen.

Gemäß einer weiteren Ausführungsart weist der horizontale Steg am äußeren Ende an der Unterseite eine erste Gleitfläche auf.

Gemäß einer weiteren Ausführungsart weist die zweite Platte an den seitlichen Rändern neben der Verbindung mit dem Schnapphaken oberhalb des Hakenendes des Schnapphakens eine Aussparung auf. Hierdurch wird das Entformen des Deckels aus einem Formwerkzeug beim Spritzgießen erleichtert.

Gemäß einer weiteren Ausführungsart weist die Mikroplatte und/oder der Deckel mindestens ein Dichtelement auf, das den Rand jeder Öffnung umlaufend abdichtet, wenn der Deckel Schnappverbindungen mit der Mikroplatte aufweist. Hierdurch wird der dichte Verschluss der Gefäße durch den Deckel in der Abdichtposition verbessert. Das Dichtelement ist beispielsweise elastisches Flachmaterial oder eine elastische Materialschicht an der Unterseite des Deckels, das sämtliche Öffnungen der Mikroplatte abdichtet, wenn der Deckel auf die Mikroplatte aufgeschnappt ist.

Gemäß einer weiteren Ausführungsart weist der Deckel an der Unterseite der zweiten Platte in Reihen und Spalten angeordnete Dichtelemente (Abdeckungen) auf, von denen jedes an einem inneren Rand einer der Öffnungen anliegt, wenn der Deckel mit der Mikroplatte verschnappt ist. Hierdurch wird die abdichtende Anlage des Deckels an den Öffnungen der Gefäße in der Abdichtposition weiter verbessert. Die Dichtelemente haben beispielsweise eine kreisrunde oder eine kreisringförmige Grundfläche und liegen umlaufend abdichtend am Rand der Öffnungen in der ersten Platte und/oder angrenzend an den Öffnungen der Innenwand der Gefäße an.

Gemäß einer weiteren Ausführungsart stehen die Dichtelemente von der Unterseite des Deckels nach unten vor.

Gemäß einer weiteren Ausführungsart ist jedes Dichtelement kuppelförmig (z..B. halbkugelförmig) oder kegelförmig. Hierdurch wird die Abdichtung der Öffnungen der Gefäße durch den Deckel in der Abdeckposition weiter verbessert.

Gemäß einer weiteren Ausführung ist das Raster der Abdeckungen bombiert. Hierbei stehen die inneren Abdeckungen höher als außenstehende Abdeckungen. Dies ist für die Abdichtung eines Rasters von Abdeckungen von Vorteil, da so ein verformtes Raster eben anliegen kann. Anders ausgedrückt geben die am meisten bzw. zuerst verformten Bereiche in der Mitte des Rasters nach und kommt erst dann der Kontakt zu den später verformten Bereichen am Rand zustande.

Gemäß einer weiteren Ausführungsart weist die erste Platte zwischen den seitlichen Rändern mindestens eine weitere Schnappöffnung auf, weist der Deckel zwischen den beiden seitlichen Rändern der zweiten Platte mindestens einen nach unten vorstehenden weiteren Schnapphaken auf, wobei der weitere Schnapphaken in die weitere Schnappöffnung eingeführt und hinter dieser an der Unterseite der ersten Platte verschnappt ist, wenn der Deckel mit der zweiten Platte an der Oberseite der ersten Platte der Mikroplatte anliegt, und der weitere Schnapphaken mindestens eine weitere erste Gleitfläche aufweist, und/oder die Entriegelungsvorrichtung zum Lösen der Schnappverbindungen eine weitere zweite Gleitfläche aufweist, die ausgebildet ist, beim Aufsetzen der Mikroplatte mit dem aufgeschnappten Deckel unter Aufhebung der Schnappverbindung auf der weiteren ersten Gleitfläche abzugleiten. Hierdurch für die abdichtende Anlage des Deckels an der Mikroplatte in der Abdeckposition weiter verbessert. Weitere Schnapphaken, die nicht am Rand, sondern zwischen den Gefäßen angeordnet sind, können die Haltekraft und die gleichmäßige Verteilung der Verrastungswirkung unterstützen. Die Schnapphaken des Deckels können nicht nur randständig, sondern auch zwischen den Wells angeordnet sein. Hierdurch können die Abdeckungen besser gegen die Ränder der Öffnungen an der Oberseite der ersten Platte gedrückt werden und zuverlässiger abdichten. Hierfür müssen in der ersten Platte weitere Schnappöffnungen (Durchbrüche) vorhanden sein, in denen auch die weiteren Schnapphaken verrasten können. Das Prinzip von Verrrastung mittels Schnapphaken und Entrastung durch Verdrängung mittels Gleitflächen am Entriegelungsblock wird auch hier genutzt.

Gemäß einer weiteren Ausführungsart weist die Entriegelungsvorrichtung einen rechteckigen Rahmen auf, werden die Stützvorsprünge von Rahmenleisten des Rahmens gebildet und ist die Aufnahme von den Innenseiten des Rahmens gebildet. Hierdurch wird eine konstruktiv besonders einfache Ausführung ermöglicht.

Gemäß einer weiteren Ausführungsart umfasst das Set mindestens eines der nachfolgenden Merkmale:
- die Mikroplatte ist aus einem einzigen oder aus mehreren Kunststoffen hergestellt, wobei gegebenenfalls der Rahmen der Mikroplatte aus einem anderen Kunststoff als die Gefäße besteht,
- der Deckel ist aus einem einzigen oder aus mehreren Kunststoffen hergestellt, wobei gegebenenfalls der zweite Rahmen aus einem anderen Kunststoff als die Dichtung besteht,
- die Entriegelungsvorrichtung besteht aus einem einzigen oder aus mehreren Kunststoffen, aus Metall oder aus einer Kombination von Kunststoff oder Metall.

Gemäß einer weiteren Ausführungsart ist die Mikroplatte und/oder der Deckel und/oder die Entriegelungsvorrichtung spritzgegossen. Gemäß einer weiteren Ausführungsart ist die Mikroplatte und/oder der Deckel im Einkomponenten- oder Mehrkomponenten-Spritzgießverfahren hergestellt.

Gemäß einer weiteren Ausführungsart ist die Mikroplatte und/oder der Deckel und/oder die Entriegelungsvorrichtung vakuumgeformt und/oder aus spritzgegossenen und vakuumgeformten Komponenten zusammengesetzt und/oder heißgeprägt.

Gemäß einer weiteren Ausführungsart haben die Gefäße ein Volumen von jeweils 0,5, 1,0, 1,2, 1,5, 1,8, 2,0 oder 2,5 ml.

Gemäß einer weiteren Ausführungsart weist die Mikroplatte 12, 24, 48, 96 oder 384 Gefäße auf.

Gemäß einer weiteren Ausführungsart ist die Mikroplatte eine Mikroplatte gemäß ANSI/SLS mit 96, 384 oder 1.536 Gefäßen oder eine Deepwell-Platte.

Die Erfindung wird nachfolgend anhand der anliegenden Zeichnungen eines Ausführungsbeispiels näher erläutert. In den Zeichnungen zeigen:
- Fig. 1: eine Mikroplatte mit verschnapptem Deckel in einer Perspektivansicht schräg von oben;
- Fig. 2: die Mikroplatte in einer Perspektivansicht schräg von unten;
- Fig. 3: vergrößertes Detail von Fig. 2;
- Fig. 4: der Deckel in einer Perspektivansicht schräg von oben;
- Fig. 5: der Deckel in einer Perspektivansicht schräg von unten;
- Fig. 6: die Mikroplatte mit dem verschnappten Deckel in einer teilweisen Seitenansicht und in einem Teilschnitt in Längsrichtung;
- Fig. 7: die Mikroplatte mit dem verschnappten Deckel in einem vergrößerten Teilschnitt;
- Fig. 8: eine Entriegelungsvorrichtung in einer Perspektivansicht;
- Fig. 9: die Entriegelungsvorrichtung mit aufgesetzter Mikroplatte und damit verschnapptem Deckel in einem Vertikalschnitt;
- Fig. 10: die Mikroplatte und der Deckel vor dem Verschnappen in einer perspektivischen Detailansicht schräg von oben;
- Fig. 11: die Mikroplatte und der damit verschnappte Deckel in einer perspektivischen Detailansicht schräg von oben;
- Fig. 12: die Mikroplatte und der damit verschnappte Deckel auf der Entriegelungsvorrichtung beim Auflösen der Schnappverbindung in einer Perspektivansicht schräg von oben.

In dieser Anmeldung beziehen sich die Angaben "horizontal" und "vertikal" sowie "oben" und "unten" auf eine Anordnung der Mikroplatte mit dem von der ersten Platte abgewandten Rand der Seitenwand auf einem horizontalen Untergrund und Deckel auf der Oberseite der Mikroplatte.

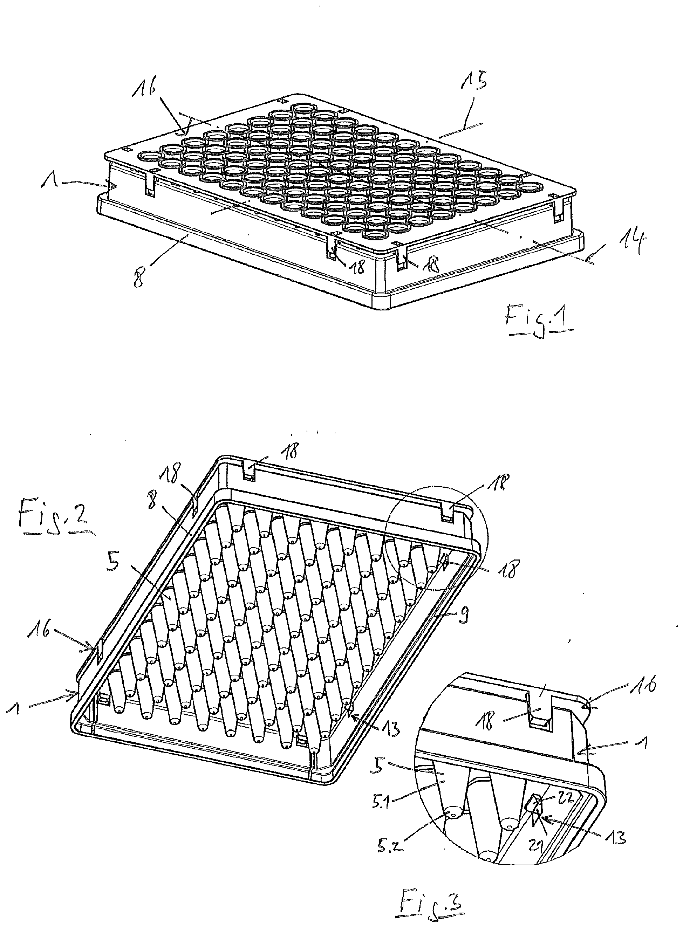

Gemäß Fig. 1 bis 3 umfasst eine Mikroplatte 1 eine rechteckige erste Platte 2 und eine vom äußeren Rand 3 der ersten Platte 2 nach unten vorstehende, umlaufende Seitenwand 4 mit vier Seiten 4.1 bis 4.4. Die erste Platte 2 und die Seitenwand 4 werden auch als "Rahmen" bezeichnet.

In der ersten Platte 2 sind 96 Gefäße 5 in acht Reihen und zwölf Spalten angeordnet, die oben mit der ersten Platte 2 verbunden sind, von der Unterseite der ersten Platte 2 nach unten vorstehen und an der Oberseite der ersten Platte 2 eine Öffnung 6 aufweisen.

Jede Öffnung 6 ist von einer kleinen Einfassung 7 umgeben, die von der Oberseite der ersten Platte 2 nach oben vorsteht.

Die Gefäße 5 weisen eine nach unten sich verjüngende Form auf. Sie haben einen oben mit der ersten Platte 2 verbundenen kegelförmigen Gefäßabschnitt 5.1 mit nach unten sich verringerndem Durchmesser und einen ersten nach unten gewölbten, kugelschalenförmigen Gefäßboden 5.2.

Die Seitenwand 4 hat am unteren Rand einen nach außen vorstehenden Flansch 8. Dieser hat im Vertikalschnitt ein umgedrehtes L-Profil und bildet eine nach außen und nach unten vorstehende Erweiterung 9 der Seitenwand 4.

Die Mikroplatte 1 weist am Rand der ersten Platte 2 Schnappöffnungen 10 auf. Jede Schnappöffnung 10 ist zu einem Teil 10.1 im seitlichen Randbereich der ersten Platte 2 und zu einem weiteren Teil 10.2 im oberen Randbereich der Seitenwand 4 angeordnet. Der Teil 10.1 der Schnappöffnung 10, der im seitlichen Randbereich der ersten Platte 2 ausgebildet ist, weist eine parallel zum äußeren Rand der ersten Platte verlaufende obere Begrenzungsfläche 11.1 auf. Von den seitlichen Enden jeder oberen Begrenzungsfläche 11.1 erstrecken sich senkrecht zu dieser obere seitliche Begrenzungsflächen 11.2, 11.3 bis zum äußeren Rand der ersten Platte 2.

Der weitere Teil 10.2 der Schnappöffnungen 10, der im oberen Randbereich der Seitenwand 4 angeordnet ist, weist eine parallel zum oberen Rand der Seitenwand 4 verlaufende untere Begrenzungsfläche 12.1 auf. Von den äußeren Enden jeder unteren Begrenzungsfläche 12.1 aus erstrecken sich vertikal nach oben untere seitliche Begrenzungsflächen 12.2, 12.3 des weiteren Teils 10.2 der Schnappöffnungen 10 bis zum oberen Rand der Seitenwand 4.

Die obere Begrenzungsfläche 11.1 fluchtet mit einer Innenseite 13 der Seitenwand 4, die an die untere Begrenzungsfläche 12.1 angrenzt, oder ist bezüglich dieser Innenseite 13 nach innen versetzt.

An jeder langen Seite der ersten Platte 2 sind zwei Schnappöffnungen 10 jeweils in der Nähe einer Ecke der Seitenwand 4 angeordnet. An den langen Seiten der ersten Platte 2 sind die Schnappöffnungen 10 symmetrisch bezüglich einer Längsmittelachse 14 der ersten Platte 2 angeordnet.

An jeder kurzen Seite der ersten Platte 2 sind zwei Schnappöffnungen 10 jeweils in der Nähe einer Ecke der Seitenwand 4 angeordnet. An den kurzen Seiten der ersten Platte 2 sind die Schnappöffnungen 10 symmetrisch bezüglich einer Quermittelachse 15 der ersten Platte 2 angeordnet.

Die Mikroplatte 1 ist aus einem einzigen oder mehreren Kunststoffen hergestellt, wobei gegebenenfalls die erste Platte 2 und die Seitenwand 4 aus einem anderen Kunststoff als die Gefäße 5 bestehen. Beispielsweise ist die Mikroplatte insgesamt aus Polystyrol, Polycarbonat oder Polypropylen hergestellt oder bestehen erste Platte 2 und Seitenwand 4 aus Polycarbonat und die Gefäße 5 aus Polypropylen, Silikon oder Flüssig-Silikonkautschuk.

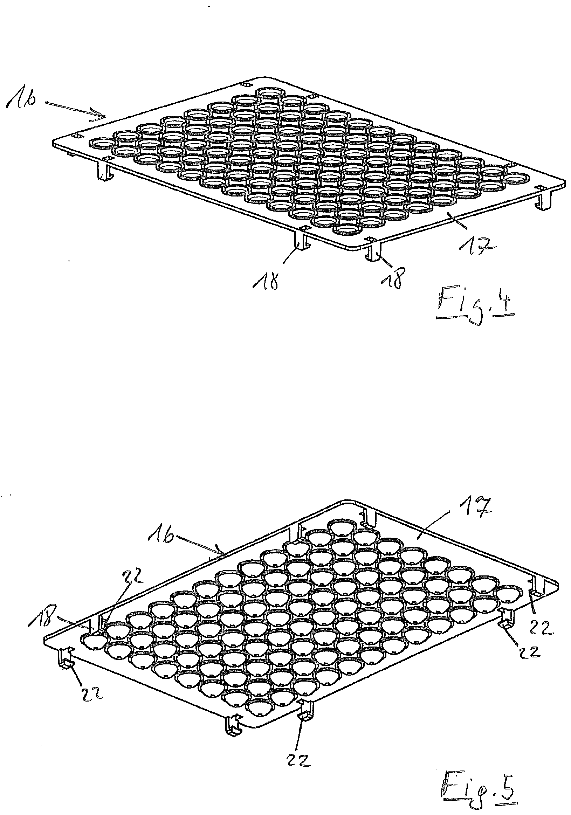

Gemäß Fig. 1 bis 3 ist auf der Mikroplatte 1 ein Deckel 16 angeordnet, der in den Fig. 4 und 5 gezeigt ist. Der Deckel 16 weist eine rechteckige zweite Platte 17 auf, die deckungsgleich zu der ersten Platte 2 ausgebildet ist.

Der Deckel 16 weist Schnapphaken 18 auf, die vom oberen äußeren Rand 19 der zweiten Platte 17 nach unten vorstehen.

Jeder Schnapphaken 18 weist einen vertikalen Steg 20 auf, der am oberen Ende mit dem oberen äußeren Rand 19 der zweiten Platte 17 verbunden ist. Ferner weist jeder Schnapphaken 18 am unteren Ende des vertikalen Steges 20 einen nach innen, d.h unterhalb der zweiten Platte 17, vorstehenden horizontalen Steg 21 auf. Der horizontale Steg 21 weist am äußeren Ende an der Unterseite eine zur Vertikalen geneigte erste Gleitfläche 22 auf.

Der Deckel 16 hat an den beiden langen Seiten der zweiten Platte 17 jeweils zwei Schnapphaken 18, wobei jeder Schnapphaken 18 an einer Ecke der zweiten Platte 17 angeordnet ist. Die beiden Schnapphaken 18 an den beiden langen Seiten der zweiten Platte 17 sind symmetrisch bezüglich einer Längsmittelachse 14 der zweiten Platte 17 angeordnet.

Auch an den beiden kurzen Seiten der zweiten Platte 17 weist der Deckel 16 jeweils zwei Schnapphaken 18 auf, von denen jeder nahe einer Ecke der zweiten Platte 17 angeordnet ist. Die Schnapphaken 18 an den beiden kürzeren Seiten sind symmetrisch bezüglich einer Quermittelachse 15 der zweiten Platte 17 angeordnet.

Wie insbesondere aus Fig. 5 ersichtlich, sind bei den symmetrisch bezüglich der Längsmittelachse 14 bzw. der Quermittelachse 15 angeordnete Schnapphaken 18 die ersten Gleitflächen 22 jeweils an einander zugewandten Innenseiten der Schnapphaken 18 angeordnet und sind dieselben ersten Gleitflächen 22 nach oben aufeinander zugeneigt. Infolgedessen schließen die ersten Gleitflächen 22 an den einander zugewandten Innenseiten der Schnapphaken 18 einen spitzen Winkel miteinander ein, dessen Scheitel oberhalb der zweiten Platte 17 angeordnet ist.

Die zweite Platte 17 weist oberhalb des horizontalen Steges 22 jedes Schnapphakens 18 eine Aussparung 23 auf. Hierdurch wird das Entformen des Deckels 16 aus einem Formwerkzeug beim Spritzgießen erleichtert.

Gemäß Fig. 4 bis 6 weist der Deckel 16 an der Unterseite der zweiten Platte 17 in Reihen und Spalten angeordnete Dichtelemente 24 auf. Jedes Dichtelement 24 ist kuppelförmig (kugelschalenförmig) und in einem kreisrunden Loch 25 in der zweiten Platte 17 gehalten. Zum Halten am Rand des Loches weist jedes Dichtelement 24 am oberen Rand einen nach außen vorstehenden, umlaufenden oberen Flansch 26 und darunter und in einem Abstand davon einen außen vorstehenden, umlaufenden unteren Flansch 27 auf. Zwischen dem oberen Flansch 26 und dem unteren Flansch 27 jedes Dichtelementes 24 greift der Rand an einem Loch 25 der zweiten Platte 17 ein, wodurch das Dichtelement 24 an der zweiten Platte 17 gehalten ist.

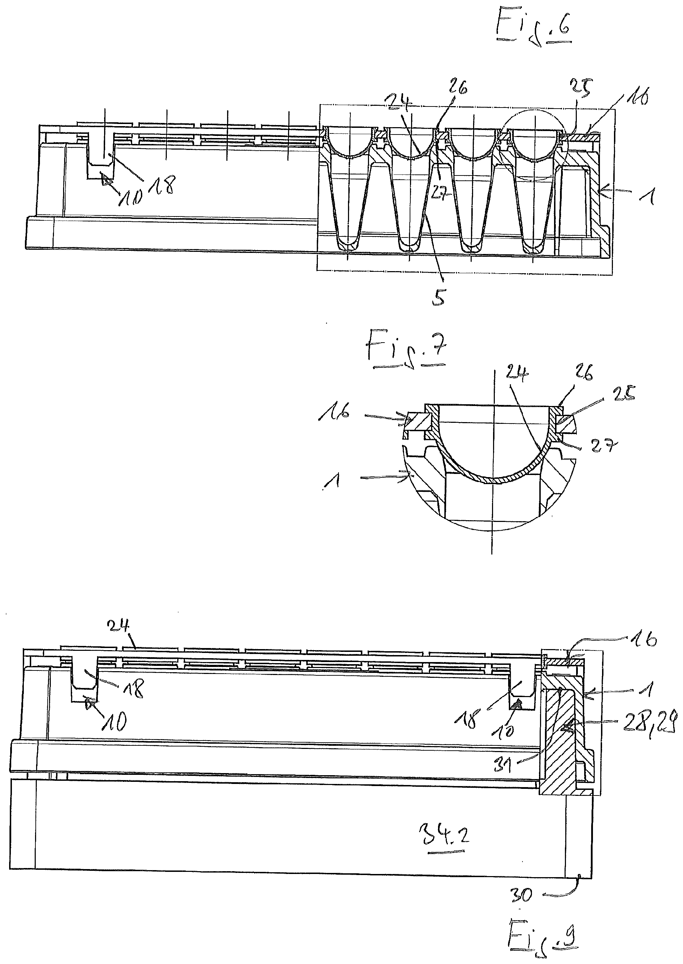

96 Dichtelemente 24 sind in der zweiten Platte 17 in acht Reihen und 12 Spalten angeordnet, wobei die Anordnung der Dichtelemente 17 der Anordnung der Gefäße 5 in der Mikroplatte 1 entspricht. Die Dichtelemente 17 sind so ausgebildet, dass jedes Dichtelement 17 teilweise in ein Gefäß 5 der Mikroplatte 1 einsetzbar ist, wobei das Dichtelement 17 am oberen Rand des Gefäßes 5 abdichtend an seiner Innenseite anliegt, wie in Fig. 6 und 7 gezeigt.

Der Deckel 16 ist durch Spritzgießen hergestellt. Das Ausführungsbeispiel mit den Dichtelementen 24 ist im Mehrkomponenten-Spritzgießverfahren hergestellt, wobei in einem ersten Schritt die zweite Platte 17 gespritzt und in einem zweiten Schritt die Dichtelemente 24 an die zweite Platte 17 angespritzt werden können. Die zweite Platte 17 wird beispielsweise aus Polystyrol, Polycarbonat oder Polypropylen hergestellt und die Dichtelemente 24 aus Polypropylen, Silikon oder Flüssig-Silikonkautschuk.

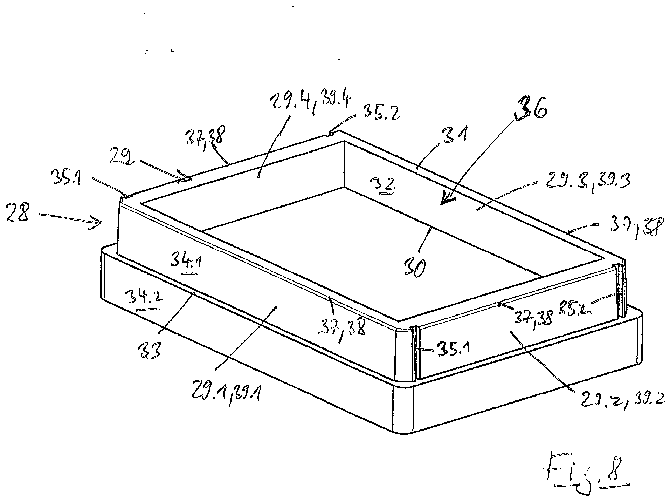

Gemäß Fig 8 weist eine Entriegelungsvorrichtung 28 einen rechteckigen Rahmen 29 auf, der vier Rahmenleisten 29.1 bis 29.4 umfasst, die an Ecken des Rahmens 29 miteinander verbunden sind. Der untere Rand des Rahmens 29 weist eine horizontale ebene Lagerfläche 30 zum Aufsetzen des Rahmens 29 auf einen horizontalen Untergrund auf. Oben hat der Rahmen eine horizontale ebene Stützfläche 21 zum Aufsetzen einer Mikroplatte 1.

An den Innenseiten weisen die Rahmenleisten jeweils eine ebene Innenfläche 32 auf.

An der Außenseite weist der Rahmen 29 einen etwa auf halber Höhe umlaufenden Absatz 33 auf.

Unterhalb des Absatzes 33 und oberhalb des Absatzes 33 weisen die Rahmenleisten 29.1 bis 29.4 jeweils eine ebene Außenfläche 34.1, 34.2 auf.

Ferner weist der Rahmen 29 an den schmalen Rahmenleisten 29.1, 29.2 auf der Außenseite oberhalb des Absatzes jeweils zwei vertikalen Nuten 35.1, 35.2 auf, wobei jede vertikale Nut 35.1, 35.2 neben einer Ecke des Rahmens 29 angeordnet ist.

Der Rahmen 29 umschließt eine Aufnahme 36, die von den Innenseiten der Rahmenleisten 29.1 bis 29.4 begrenzt wird.

Der Rahmen 29 weist am oberen Rand außen eine umlaufende Fase 37 auf, die zweite Gleitflächen 38 bildet. Die zweiten Gleitflächen 38 schließen einen Winkel zur Vertikalen ein. Auf einander gegenüberliegenden Rahmenleisten 29.1 bis 29.3 sind die zweiten Gleitflächen 38 so angeordnet, dass sie nach oben aufeinander zugeneigt sind. Infolgedessen schließen sie einen spitzen Winkel mit einem Scheitel oberhalb des Rahmens 29 miteinander ein.

Die Rahmenleisten 29.1 bis 29.4 bilden Stützvorsprünge 39.1 bis 39.4 zum Aufsetzen der Mikroplatte 1.

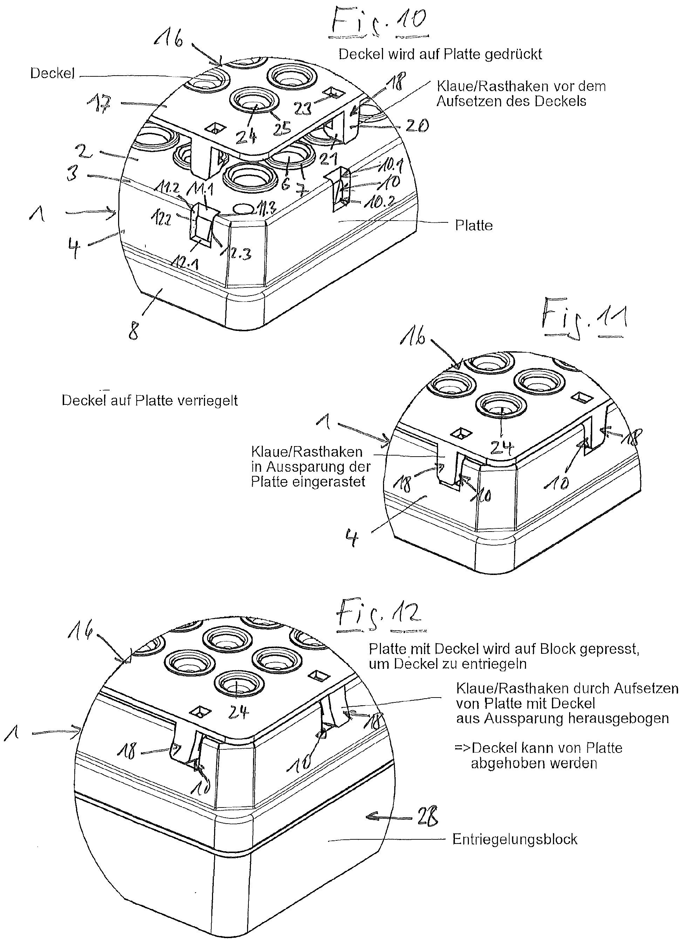

Gemäß Fig. 10 und 11 ist der Deckel 16 auf die Mikroplatte 1 aufsetzbar, wobei die Schnapphaken 18 Schnappverbindungen an den Schnappöffnungen 10 bilden, bei denen die horizontalen Stege 22 die erste Platte 2 an den seitlichen Rändern untergreifen. Wenn der Deckel 16 auf die Mikroplatte 1 aufgesetzt ist, greifen die Dichtelemente 24 abdichtend in die Öffnungen 6 der Gefäße 5 ein, wie in den Fig. 6 und 7 gezeigt. In dieser Stellung wird der Deckel 16 durch die Schnapphaken 18 an der Mikroplatte 1 gesichert.

Der Deckel 16 wird von oben auf die Mikroplatte 1 aufgesetzt. Hierbei tauchen die Schnapphaken 18 am Deckel 16 in die Schnappöffnungen 10 der Mikroplatte 1 ein. Die Schnapphaken 18 schnappen formschlüssig hinter den Rändern der Schnappöffnungen 10 ein und halten den Deckel 16 auf der Mikroplatte 1 fest. Bevor die Schnapphaken 18 einschnappen, liegen die Dichtelemente 24 dichtend im Oberbereich an den Innenseiten der Gefäße 5 an und decken diese ab.

Der Deckel 16 kann wieder entfernt werden, indem die Mikroplatte 1 mit dem aufgeschnappten Deckel 16 auf die Entriegelungsvorrichtung 28 gesetzt wird. Die Außenseite und Oberseite der Entriegelungsvorrichtung 28 ist als negativ zu der Innenseite der Mikroplatte 1 ausgebildet. Infolgedessen ist die Mikroplatte 1 mit einem damit aufgeschnappten Deckel 16 auf den Rahmen 29 aufsetzbar, sodass der Rahmen 29 von unten in die umlaufende Seitenwand 4 der Mikroplatte eingreift und die Gefäße 5 in die Aufnahme 36 eintauchen. Beim Aufsetzen der Mikroplatte 1 auf die Entriegelungsvorrichtung 28 können vertikale Rippen zur konstruktiven Versteifung an der Innenseite der Seitenwand 4 in die Nuten 35.1, 35.2 des Rahmens 29 eingreifen.

Am Ende der Aufsetzbewegung gleiten die ersten Gleitflächen 22 der Schnapphaken 18 auf den zweiten Gleitflächen 38 des Rahmens 29 ab und werden hierdurch die Schnapphaken 18 nach außen gebogen. Hierdurch werden die Schnappverbindungen aufgehoben, sodass der Deckel 16 von der Mikroplatte 1 abgehoben und entfernt werden kann. Dies ist in der Fig. 12 gezeigt. Hierbei kann der Deckel 16 von dem Rahmen 29 nach oben gedrückt werden. Nach dem Auflösen der Schnappverbindungen ist der Deckel 16 von der Mikroplatte 1 abziehbar.

Das Aufsetzen und Verschnappen des Deckels 16 mit der Mikroplatte 1 sowie das Lösen der Schnappverbindungen und Abnehmen des Deckels 16 von der Mikroplatte 1 kann automatisiert durchgeführt werden, beispielsweise in einem Laborautomaten oder Dosierautomaten.

Die Mikroplatte und der Deckel 16 sind durch Greifer eines automatisierten Systems (z. B. Epmotion der Anmelderin) handhabbar.

Der Deckel 16 kann ähnlich wie die Mikroplatte 1 aufgrund des verwendeten Kunststoffes und gegebenenfalls durch konstruktive Versteifung (Verrippung oder Rahmen) formstabil und gut handhabbar sein
Die Dichtelemente 24 werden vorzugsweise aus einem entsprechend weichen bzw. elastischen Material gebildet. Dies ist beispielsweise ein thermoplastisches Material, z.B. ein weiches PP, PE oder TPE. Aufgrund der Nachgiebigkeit der Dichtelemente 24 und ihrer Anordnung im Raster der Gefäße 5 der Mikroplatte 1 kann jedes Dichtelement 24 ein Gefäß 5 abdecken.

Der Deckel 16 mit den Dichtelementen 24 kann aus mehreren zusammengebauten Teilen bestehen oder im Zweikomponenten-Spritzguss hergestellt werden.

### Bezugszeichenliste

- 1: Mikroplatte
- 2: erste Platte
- 3: äußerer Rand
- 4: Seitenwand
- 4.1-4.4: Seiten
- 5: Gefäße
- 5.1: Gefäßabschnitt
- 5.2: Gefäßboden
- 6: Öffnung
- 7: Einfassung
- 8: Flansch
- 9: Erweiterung
- 10: Schnappöffnung
- 10.1: Teil der Schnappöffnung
- 10.2: weiterer Teil der Schnappöffnung
- 11.1: obere Begrenzungsfläche
- 11.2, 11.3: obere seitliche Begrenzungsfläche
- 12.1: untere Begrenzungsfläche
- 12.2, 12.3: untere seitliche Begrenzungsfläche
- 13: Innenseite
- 14: Längsmittelachse
- 15: Quermittelachse
- 16: Deckel
- 17: zweite Platte
- 18: Schnapphaken
- 19: oberer äußerer Rand
- 20: vertikaler Steg
- 21: horizontaler Steg
- 22: erste Gleitfläche
- 23: Aussparung
- 24: Dichtelement
- 25: Loch
- 26: oberer Flansch
- 27: unterer Flansch
- 28: Entriegelungsvorrichtung
- 29: Rahmen
- 29.1-29.4: Rahmenleiste
- 30: Lagerfläche
- 31: Stützfläche
- 32: Innenfläche
- 33: Absatz
- 34.1, 34.2: Außenfläche
- 35.1, 35.2: Nut
- 36: Aufnahme
- 37: Fase
- 38: zweite Gleitfläche
- 39.1-39.4: Stützvorsprung

## Patentansprüche

1. Set, umfassend:
• eine Mikroplatte (1),
• mit einer rechteckigen ersten Platte (2),
• einer Vielzahl Gefäße (5), die in Reihen und Spalten angeordnet sind, oben mit der ersten Platte (2) verbunden sind, von der Unterseite der ersten Platte nach unten vorstehen und an der Oberseite der ersten Platte eine Öffnung (6) aufweisen, und
• Schnappöffnungen (10) an mindestens zwei einander gegenüberliegenden seitlichen Rändern der ersten Platte (2),
• einen Deckel (16),
• mit einer zweiten Platte (17) zum Aufsetzen auf die Oberseite der ersten Platte (2) und Abdecken der Öffnungen (6) der Gefäße (5) und
• von der Unterseite der zweiten Platte (17) vorstehenden Schnapphaken (18) an mindestens zwei einander gegenüberliegenden seitlichen Rändern der zweiten Platte (17), wobei der Deckel (16) in einer Abdeckposition mit der zweiten Platte (17) auf die Oberseite der ersten Platte (2) aufsitzt, die Öffnungen (6) der Gefäße (5) abdeckt und mit den Schnapphaken (18) unter Ausbildung von Schnappverbindungen in die Schnappöffnungen (10) eingeschnappt ist, und
• eine Entriegelungsvorrichtung (28) zum Lösen der Schnappverbindungen zwischen Mikroplatte (1) und Deckel (16),
• mit mindestens zwei hochstehenden Stützvorsprüngen (39.1-39.4) und mindestens einer Aufnahme (36) dazwischen zum Aufsetzen einer Mikroplatte (1) mit mindestens zwei einander gegenüberliegenden Randbereichen der ersten Platte (2) auf die Stützvorsprünge unter Eingriff der Gefäße (5) in die mindestens eine Aufnahme (36) und
• erste Gleitflächen (22) an einander zugewandten Innenseiten der Schnapphaken (18) an einander gegenüberliegenden Rändern der ersten Platte (2) und zweite Gleitflächen (38) an voneinander abgewandten Außenseiten der Stützvorsprünge (39.1 - 39.4) auf einander gegenüberliegenden Seiten der Aufnahme (36), wobei die ersten Gleitflächen (22) an einander zugewandten Innenseiten der Schnapphaken (18) nach oben aufeinander zugeneigt sind und/oder die zweiten Gleitflächen (38) an voneinander abgewandten Außenseiten der Stützvorsprünge (39.1-39.4) nach oben aufeinander zugeneigt sind und beim Aufsetzen der Mikroplatte (1) mit dem aufgeschnappten Deckel (16) auf die Entriegelungsvorrichtung (28) durch Abgleiten der zweiten Gleitflächen (38) auf den ersten Gleitflächen (22) die Schnapphaken (18) nach außen ausgelenkt und die Schnappverbindungen aufgehoben werden.

2. Set nach Anspruch 1, bei dem jede Schnappöffnung (10) zumindest teilweise in den beiden seitlichen Rändern der ersten Platte (2) angeordnet ist.

3. Set nach Anspruch 1 oder 2, bei der die Mikroplatte (1) eine vom äußeren Rand der ersten Platte (2) nach unten vorstehende, umlaufende Seitenwand (4) umfasst und jede Schnappöffnung (10) zumindest teilweise im oberen Randbereich der Seitenwand (4) ausgebildet ist.

4. Set nach einem der Ansprüche 1 bis 3, bei dem jede Schnappöffnung (10) zu einem Teil (10.1) im seitlichen Randbereich der ersten Platte (2) und zu einem weiteren Teil (10.2) im oberen Randbereich der Seitenwand (4) angeordnet ist.

5. Set nach Anspruch 4, bei dem der Teil (10.1) der Schnappöffnung (10), der im seitlichen Randbereich der ersten Platte (2) ausgebildet ist, eine parallel zum äußeren Rand der ersten Platte verlaufende obere Begrenzungsfläche (11.1) aufweist, die mit der Innenseite (13) der Seitenwand (4) fluchtet oder bezüglich dieser nach innen versetzt ist, die an eine untere Begrenzungsfläche (12.1) des Teils (10.2) der Schnappöffnung (10) im oberen Randbereich der Seitenwand (4) angrenzt.

6. Set nach einem der Ansprüche 1 bis 5, bei dem die Mikroplatte (1) an den beiden langen Rändern der ersten Platte (2) jeweils zwei Schnappöffnungen (10) aufweist und bei dem der Deckel (16) an den beiden langen Rändern der zweiten Platte (17) jeweils zwei Schnapphaken (18) aufweist.

7. Set nach einem der Ansprüche 1 bis 6, bei dem die Mikroplatte (1) an den beiden kurzen Rändern der ersten Platte (2) jeweils zwei Schnappöffnungen (10) aufweist und der Deckel (16) an den beiden kurzen Rändern jeweils zwei Schnapphaken (18) aufweist.

8. Set nach einem der Ansprüche 1 bis 7, bei dem jeder Schnapphaken (18) einen vertikalen Steg (/20) und am unteren Ende des vertikalen Steges einen nach innen vorstehenden horizontalen Steg (21) aufweist.

9. Set nach Anspruch 8, bei dem der horizontale Steg (21) am äußeren Ende an der Unterseite eine erste Gleitfläche (22) aufweist.

10. Set nach einem der Ansprüche 1 bis 9, bei dem die zweite Platte (17) an den seitlichen Rändern neben der Verbindung mit dem Schnapphaken (18) oberhalb des Hakenendes des Schnapphakens eine Aussparung (23) aufweist.

11. Set nach einem der Ansprüche 1 bis 10, dem die Mikroplatte (1) und/oder der Deckel (16) mindestens ein Dichtelement (24) aufweist, das den Rand jeder Öffnung (6) umlaufend abdichtet, wenn der Deckel (16) mit der Mikroplatte (1) verschnappt ist.

12. Set nach einem der Ansprüche 1 bis 11, bei dem der Deckel (16) an der Unterseite der zweiten Platte in Reihen und Spalten angeordnete Dichtelemente (24) aufweist, von denen jedes an einem inneren Rand einer der Öffnungen (6) anliegt, wenn der Deckel (16) mit der Mikroplatte (1) verschnappt ist.

13. Set nach einem der Ansprüche 1 bis 12, bei dem die Mikroplatte (1) in der ersten Platte (2) zwischen den seitlichen Rändern mindestens eine weitere Schnappöffnung (10) aufweist, der Deckel (16) zwischen den beiden seitlichen Rändern der zweiten Platte (17) mindestens einen nach unten vorstehenden weiteren Schnapphaken (18) aufweist, wobei weitere Schnapphaken (18) in die weitere Schnappöffnung (10) eingeführt und hinter dieser an der Unterseite der ersten Platte (2) verschnappt ist, wenn der Deckel (16) mit der zweiten Platte (17) an der Oberseite der ersten Platte (2) der Mikroplatte (1) anliegt, und die Entriegelungsvorrichtung (28) und/oder der weitere Schnapphaken (18) mindestens eine weitere zweite Gleitfläche (38) aufweisen, die ausgebildet ist, beim Aufsetzen der Mikroplatte (1) mit dem aufgeschnappten Deckel (16) unter Aufhebung der Schnappverbindung auf der weiteren ersten Gleitfläche (22) abzugleiten.

14. Set nach einem der Ansprüche 1 bis 13, bei dem die Entriegelungsvorrichtung (28) einen rechteckigen Rahmen (29) aufweist, die Stützvorsprünge (39.1 bis 39.4) von Rahmenleisten (29.1 - 29.4) des Rahmens gebildet werden, die Aufnahme (36) von den Innenseiten der Rahmenleisten (29.1 - 29.4) begrenzt wird und die Stützfläche (31) von den oberen Stirnflächen der Rahmenleisten gebildet wird.

15. Set nach einem der Ansprüche 1 bis 14 umfassend mindestens eines der nachfolgenden Merkmale:
• die Mikroplatte (1) ist aus einem einzigen oder aus mehreren Kunststoffen hergestellt, wobei gegebenenfalls ihr Rahmen aus einem anderen Kunststoff als die Gefäße (5) besteht,
• der Deckel (16) ist aus einem einzigen oder aus mehreren Kunststoffen hergestellt, wobei gegebenenfalls die zweite Platte (17) aus einem anderen Kunststoff als das Dichtelement (24) besteht,
• die Entriegelungsvorrichtung (28) besteht aus einem einzigen oder mehreren Kunststoffen, aus Metall oder aus einer Kombination von Kunststoff und Metall,
• die Mikroplatte (1) und/oder der Deckel (16) und/oder die Entriegelungsvorrichtung (28) ist spritzgegossen.

## Claims

1. A set, comprising:
• a microplate (1),
• having a rectangular first plate (2),
• a large number of wells (5) which are arranged in rows and columns and connected at the top to the first plate (2), project downward from the underside of the first plate, and have an opening (6) on the upper side of the first plate, and
• snap openings (10) on at least two mutually opposing lateral edges of the first plate (2),
• a lid (16),
• having a second plate (17) for placement onto the upper side of the first plate (2) and for covering the openings (6) of the wells (5) and
• snap hooks (18) projecting from the underside of the second plate (17) on at least two mutually opposing lateral edges of the second plate (17), wherein the lid (16) rests with the second plate (17) on the upper side of the first plate (2) in a covering position, covers the openings (6) of the wells (5), and is snapped into the snap openings (10) by means of the snap hooks (18) so as to form snap connections, and
• an unlocking device (28) for releasing the snap connections between the microplate (1) and the lid (16),
• having at least two upwardly projecting support projections (39.1-39.4) and at least one receiving portion (36) therebetween for placing a microplate (1) with at least two mutually opposing edge regions of the first plate (2) onto the support projections with engagement of the wells (5) in the at least one receiving portion (36) and
• first sliding surfaces (22) on inner sides of the snap hooks (18) that face one another on mutually opposing edges of the first plate (2) and second sliding surfaces (38) on outer sides of the support projections (39.1 - 39.4) that face away from one another on mutually opposing sides of the receiving portion (36), wherein the first sliding surfaces (22) on inner sides of the snap hooks (18) that face one another are inclined upward toward one another and/or the second sliding surfaces (38) on outer sides of the support projections (39.1-39.4) that face away from one another are inclined upward toward one another and, when placing the microplate (1) with snapped on lid (16) onto the unlocking device (28), the snap hooks (18) are deflected outward and the snap connections are released through sliding the second sliding surfaces (38) on the first sliding surfaces (22).

2. The set according to claim 1, wherein each snap opening (10) is arranged at least in part in the two lateral edges of the first plate (2).

3. The set according to claim 1 or 2, wherein the microplate (1) comprises a circumferential side wall (4) that projects downward from the outer edge of the first plate (2), and each snap opening (10) is formed at least in part in the upper edge region of the side wall (4).

4. The set according to any one of claims 1 to 3, wherein each snap opening (10) is arranged in one part (10.1) in the lateral edge region of the first plate (2) and in another part (10.2) in the upper edge region of the side wall (4).

5. The set according to claim 4, wherein the part (10.1) of the snap opening (10) that is formed in the lateral edge region of the first plate (2) comprises an upper boundary surface (11.1) that extends parallel to the outer edge of the first plate and that is flush with the inner side (13) of the side wall (4) or is offset inward relative thereto, which inner side adjoins a lower boundary surface (12.1) of the part (10.2) of the snap opening (10) in the upper edge region of the side wall (4).

6. The set according to any one of claims 1 to 5, wherein the microplate (1) comprises two snap openings (10) in each case on the two long edges of the first plate (2) and wherein the lid (16) comprises two snap hooks (18) in each case on the two long edges of the second plate (17).

7. The set according to any one of claims 1 to 6, wherein the microplate (1) comprises two snap openings (10) in each case on the two short edges of the first plate (2) and the lid (16) comprises two snap hooks (18) in each case on the two short edges.

8. The set according to any one of claims 1 to 7, wherein each snap hook (18) comprises a vertical portion (/20) and an inwardly projecting horizontal portion (21) at the lower end of the vertical portion.

9. The set according to claim 8, wherein the horizontal portion (21) comprises a first sliding surface (22) at the outer end on the underside.

10. The set according to any one of claims 1 to 9, wherein the second plate (17) comprises a cutout (23) on the lateral edges next to the connection with the snap hook (18) above the hook end of the snap hook.

11. The set according to any one of claims 1 to 10, which the microplate (1) and/or the lid (16) comprises at least one sealing element (24) which circumferentially seals the edge of each opening (6) when the lid (16) is snap-fitted to the microplate (1).

12. The set according to any one of claims 1 to 11, wherein the lid (16) comprises sealing elements (24) arranged on the underside of the second plate in rows and columns, each of which sealing elements abuts an inner edge of one of the openings (6) when the lid (16) has been snap-fitted to the microplate (1).

13. The set according to any one of claims 1 to 12, wherein, in the first plate (2), the microplate (1) comprises at least one further snap opening (10) between the lateral edges, the lid (16) comprises at least one downwardly projecting further snap hook (18) between the two lateral edges of the second plate (17), wherein further snap hook (18) is introduced into the further snap opening (10) and is snap-fitted therebehind on the underside of the first plate (2) when the lid (16), with the second plate (17), abuts the upper side of the first plate (2) of the microplate (1), and the unlocking device (28) and/or the further snap hook (18) comprises at least one further second sliding surface (38) which is designed to slide on the further first sliding surface (22) when the microplate (1) with the lid (16) snapped on is placed on so as to undo the snap connection.

14. The set according to any one of claims 1 to 13, wherein the unlocking device (28) comprises a rectangular frame (29), the support projections (39.1 to 39.4) are formed by frame strips (29.1 - 29.4) of the frame, the receiving portion (36) is delimited by the inner sides of the frame strips (29.1 - 29.4), and the support surface (31) is formed by the upper end surfaces of the frame strips.

15. The set according to any one of claims 1 to 14, comprising at least one of the following features:
• the microplate (1) is manufactured from a single or from multiple plastics materials, wherein the frame thereof optionally consists of a different plastics material than the wells (5),
• the lid (16) is manufactured from a single or from multiple plastics materials, wherein the second plate (17) optionally consists of a different plastics material than the sealing element (24),
• the unlocking device (28) consists of a single or of multiple plastics materials, of metal, or of a combination of plastics material and metal,
• the microplate (1) and/or the lid (16) and/or the unlocking device (28) is inj ection-molded.

## Revendications

1. Ensemble, comprenant :
• une microplaque (1),
• avec une première plaque rectangulaire (2),
• une pluralité de récipients (5) disposés en rangées et en colonnes, reliés à la première plaque (2) sur le haut, font saillie vers le bas à partir de la face inférieure de la première plaque et présentent une ouverture (6) sur la face supérieure de la première plaque, et
• des ouvertures d'encliquetage (10) sur au moins deux bords latéraux mutuellement opposés de la première plaque (2),
• un couvercle (16),
• avec une deuxième plaque (17) destinée à être montée sur la face supérieure de la première plaque (2) et à recouvrir les ouvertures (6) des récipients (5) et
• des crochets d'encliquetage (18) faisant saillie à partir de la face inférieure de la deuxième plaque (17) sur au moins deux bords latéraux mutuellement opposés de la deuxième plaque (17), dans lequel le couvercle (16) repose sur la face supérieure de la première plaque (2) dans une position de recouvrement avec la deuxième plaque (17), recouvre les ouvertures (6) des récipients (5) et est encliqueté avec les crochets d'encliquetage (18) dans les ouvertures d'encliquetage (10) en formant des liaisons par encliquetage, et
• un dispositif de libération (28) destiné à détacher les liaisons par encliquetage entre la microplaque (1) et le couvercle (16),
• avec au moins deux saillies d'appui (39.1 - 39.4) dressées vers le haut et au moins un logement (36) entre celles-ci pour le montage d'une microplaque (1) avec au moins deux régions de bords mutuellement opposées de la première plaque (2) sur les saillies d'appui sous engagement des récipients (5) dans l'au moins un logement (36) et
• des premières surfaces de glissement (22) sur des côtés intérieurs tournés les uns vers les autres des crochets d'encliquetage (18) sur des bords mutuellement opposés de la première plaque (2) et des deuxièmes surfaces de glissement (38) sur des côtés extérieurs détournés les uns des autres des saillies d'appui (39.1 - 39.4) sur des côtés mutuellement opposés du logement (36), dans lequel les premières surfaces de glissement (22) sont inclinées les unes vers les autres vers le haut sur des côtés intérieurs tournés les uns vers les autres des crochets d'encliquetage (18) et/ou les deuxièmes surfaces de glissement (38) sont inclinées les unes vers les autres vers le haut sur des côtés extérieurs détournés les uns des autres des saillies d'appui (39.1 - 39.4) et les crochets d'encliquetage (18) sont déviés vers l'extérieur et les liaisons par encliquetage sont supprimées lors du montage de la microplaque (1) avec le couvercle (16) encliqueté sur le dispositif de libération (28) par glissement des deuxièmes surfaces de glissement (38) sur les premières surfaces de glissement (22).

2. Ensemble selon la revendication 1, dans lequel chaque ouverture d'encliquetage (10) est au moins partiellement disposée dans les deux bords latéraux de la première plaque (2).

3. Ensemble selon la revendication 1 ou 2, dans lequel la microplaque (1) comporte une paroi latérale périphérique (4) faisant saillie vers le bas à partir du bord extérieur de la première plaque (2) et chaque ouverture d'encliquetage (10) est au moins partiellement formée dans la région de bord supérieure de la paroi latérale (4).

4. Ensemble selon l'une des revendications 1 à 3, dans lequel chaque ouverture d'encliquetage (10) est disposée en une partie (10.1) dans la région de bord latérale de la première plaque (2) et en une autre partie (10.2) dans la région de bord supérieure de la paroi latérale (4).

5. Ensemble selon la revendication 4, dans lequel la partie (10.1) de l'ouverture d'encliquetage (10) formée dans la région de bord latérale de la première plaque (2) présente une surface de délimitation supérieure (11.1) s'étendant parallèlement au bord extérieur de la première plaque, laquelle est alignée avec le côté intérieur (13) de la paroi latérale (4) ou décalée vers l'intérieur par rapport à celui-ci, laquelle est adjacente à une surface de délimitation inférieure (12.1) de la partie (10.2) de l'ouverture d'encliquetage (10) dans la région de bord supérieure de la paroi latérale (4).

6. Ensemble selon l'une des revendications 1 à 5, dans lequel la microplaque (1) présente respectivement deux ouvertures d'encliquetage (10) sur les deux bords longs de la première plaque (2) et dans lequel le couvercle (16) présente respectivement deux crochets d'encliquetage (18) sur les deux bords longs de la deuxième plaque (17).

7. Ensemble selon l'une des revendications 1 à 6, dans lequel la microplaque (1) présente respectivement deux ouvertures d'encliquetage (10) sur les deux bords courts de la première plaque (2) et le couvercle (16) présente respectivement deux crochets d'encliquetage (18) sur les deux bords courts.

8. Ensemble selon l'une des revendications 1 à 7, dans lequel chaque crochet d'encliquetage (18) présente une âme verticale (20) et une âme horizontale (21) faisant saillie vers l'intérieur à l'extrémité inférieure de l'âme verticale.

9. Ensemble selon la revendication 8, dans lequel l'âme horizontale (21) présente une première surface de glissement (22) à l'extrémité extérieure sur la face inférieure.

10. Ensemble selon l'une des revendications 1 à 9, dans lequel la deuxième plaque (17) présente un évidement (23) sur les bords latéraux à côté de la liaison avec le crochet d'encliquetage (18) au-dessus de l'extrémité de crochet du crochet d'encliquetage.

11. Ensemble selon l'une des revendications 1 à 10, dans lequel la microplaque (1) et/ou le couvercle (16) présentent au moins un élément d'étanchéité (24), lequel étanchéifie circonférentiellement le bord de chaque ouverture (6) lorsque le couvercle (16) est encliqueté avec la microplaque (1).

12. Ensemble selon l'une des revendications 1 à 11, dans lequel le couvercle (16) présente des éléments d'étanchéité (24) disposés en rangées et en colonnes sur la face inférieure de la deuxième plaque, parmi lesquels chacun s'applique sur un bord intérieur de l'une des ouvertures (6) lorsque le couvercle (16) est encliqueté avec la microplaque (1).

13. Ensemble selon l'une des revendications 1 à 12, dans lequel la microplaque (1) présente au moins une autre ouverture d'encliquetage (10) entre les bords latéraux dans la première plaque (2), le couvercle (6) présente au moins un autre crochet d'encliquetage (18) faisant saillie vers le bas entre les deux bords latéraux de la deuxième plaque (17), dans lequel l'autre crochet d'encliquetage (18) est introduit dans l'autre ouverture d'encliquetage (10) et encliqueté derrière celle-ci sur la face inférieure de la première plaque (2) lorsque le couvercle (16) s'applique avec la deuxième plaque (17) sur la face supérieure de la première plaque (2) de la microplaque (1), et le dispositif de libération (28) et/ou l'autre crochet d'encliquetage (18) présentent au moins une autre deuxième surface de glissement (38), laquelle est conçue pour glisser sur l'autre première surface de glissement (22) lors du montage de la microplaque (1) avec le couvercle encliqueté (16) sous suppression de la liaison par encliquetage.

14. Ensemble selon l'une des revendications 1 à 13, dans lequel le dispositif de libération (28) présente un cadre rectangulaire (29), les saillies d'appui (39.1 - 39.4) sont formées par des bordures de cadre (29.1 - 29.4) du cadre, le logement (36) est délimité par les côtés intérieurs des bordures de cadre (29.1 - 29.4) et la surface d'appui (31) est formée par les surfaces frontales supérieures des bordures de cadre.

15. Ensemble selon l'une des revendications 1 à 14, comportant au moins l'une des caractéristiques suivantes :
• la microplaque (1) est fabriquée à partir d'une seule ou de plusieurs matières plastiques, son cadre étant si nécessaire constitué d'une autre matière plastique que les récipients (5),
• le couvercle (16) est fabriqué à partir d'une seule ou de plusieurs matières plastiques, la deuxième plaque (17) étant si nécessaire constituée d'une autre matière plastique que l'élément d'étanchéité (24),
• le dispositif de libération (28) est constitué d'une seule ou de plusieurs matières plastiques, de métal ou d'une combinaison de matière plastique et de métal,
• la microplaque (1) et/ou le couvercle (16) et/ou le dispositif de libération (28) sont moulés par injection.
